(19) Europäisches Patentamt
European Patent Office
Office européen des brevets

(11) Numéro de publication : 0 275 221 B1

(12) # FASCICULE DE BREVET EUROPEEN

(45) Date de publication du fascicule du brevet : **17.04.91 Bulletin 91/16**

(51) Int. Cl.$^5$ : **C07D 209/08**

(21) Numéro de dépôt : **88400036.5**

(22) Date de dépôt : **08.01.88**

(54) **Nouveaux dérivés du N-(1H-indol 4-yl) benzamide ainsi que leurs sels, leur application à titre de médicaments et les compositions les renfermant.**

(30) Priorité : **09.01.87 FR 8700151**

(43) Date de publication de la demande : **20.07.88 Bulletin 88/29**

(45) Mention de la délivrance du brevet : **17.04.91 Bulletin 91/16**

(84) Etats contractants désignés : **AT BE CH DE ES FR GB GR IT LI LU NL SE**

(56) Documents cités :
**EP-A- 0 201 400**
**FR-A- 2 583 047**

(73) Titulaire : **ROUSSEL-UCLAF**
**35, boulevard des Invalides**
**F-75007 Paris (FR)**

(72) Inventeur : **Clemence, François**
**2, rue Turgot**
**F-75009 Paris (FR)**
Inventeur : **Guillaume, Jacques**
**62, rue Tixérecourt**
**F-75020 Paris (FR)**
Inventeur : **Hamon, Gilles**
**40, rue de Bagneux**
**F-92120 Montrouge (FR)**

(74) Mandataire : **Bourgouin, André et al**
**Département des Brevets ROUSSEL UCLAF**
**111, route de Noisy B.P. no 9**
**F-93230 Romainville (FR)**

EP 0 275 221 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention concerne de nouveaux dérivés du N-(1H-indole 4-yl) benzamide ainsi que leurs sels, leur application à titre de médicaments et les compositions les renfermant.

Des dérivés du N-(1H-indol 4-yl) benzamide sont décrits dans la demande de brevet européen EP A 213 984. Ces dérivés répondent à la formule générale suivante :

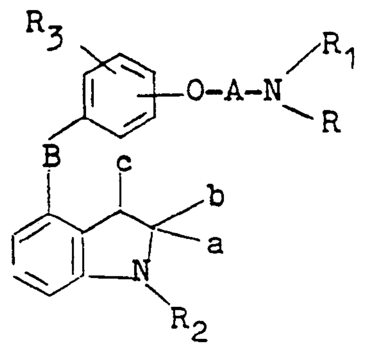

dans laquelle R et $R_1$ représentent, indépendamment l'un de l'autre, un atome d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone éventuellement substitué par 1, 2 ou 3 radicaux choisis dans le groupe constitué par les halogènes et les radicaux méthyle, éthyle, méthoxy, éthoxy, trifluoro méthyle, méthylthio, amino et nitro, ou R et $R_1$ forment ensemble un hétérocycle saturé ou insaturé pouvant renfermer un second hétéroatome choisi parmi les atomes d'oxygène, de soufre et d'azote, cet atome d'azote étant éventuellement substitué par un radical alkyle renfermant de 1 à 5 atomes de carbone, par un radical phényle, phényle substitué ou naphtyle ou par un radical aralkyle renfermant de 7 à 12 atomes de carbone, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou un radical alkyle renfermant de 1 à 5 atomes de carbone, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, A représente une chaîne $-(CH_2)_n-$ dans laquelle n peut prendre les valeurs 2, 3, 4 ou 5, ou une chaîne

$$-(CH_2)_m-\underset{\displaystyle OH}{\underset{|}{CH}}-CH_2-$$

dans laquelle m peut prendre les valeurs 1, 2 ou 3, 3, B représente une chaîne –CO-NH–ou–NH-CO–, $R_2$ représente un atome d'hydrogène, un radical alkyle linéaire renfermant de 1 à 5 atomes de carbone ou un radical alkyle ramifié renfermant de 3 à 5 atomes de carbone.

Ces composés sont décrits comme présentant d'intéressantes propriétés pharmacologiques et notamment de remarquables propriété anti-arythmiques. La présente invention concerne des composés nouveaux rentrant dans la formule générale de cette demande de brevet européen, nouveaux composés qui sont doués de propriétés anti-arythmiques particulièrement intéressantes.

La présente invention a donc pour objet des composés de formule (I) :

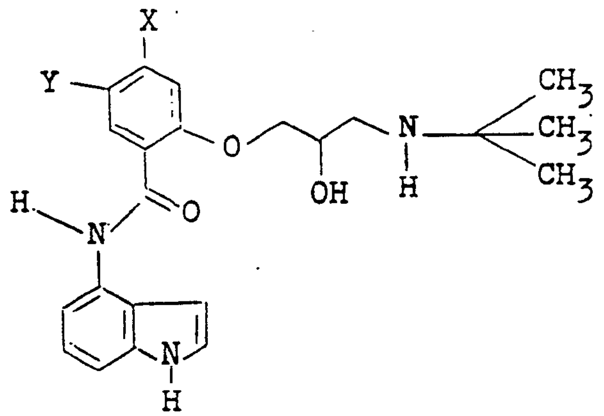

(I)

dans laquelle l'un ou l'autre des substituants X ou Y représente un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou un ou deux radicaux alkyles renfermant de 1 à 5 atomes de carbone et l'autre substituant représente un atome d'hydrogène, étant entendu que Y ne représente pas le groupement méthoxy et X un atome de chlore ainsi que leurs sels d'addition avec les acides.

Lorsque X ou Y représente un radical alkyle, il s'agit de préférence d'un radical méthyle ou éthyle, mais ils peuvent aussi représenter un radical n-propyle, isopropyle, n-butyle, isobutyle, tert-butyle ou n-pentyle.

Lorsque X ou Y représente un radical alkoxy, il s'agit de préférence d'un radical méthoxy, éthoxy, n-propoxy ou isopropoxy.

Lorsque X ou Y représente un radical amino substitué par un groupe acyle aliphatique renfermant de 2 à 5 atomes de carbone, il s'agit de préférence d'un radical amino substitué de préférence par un radical acétyle ou propionyle.

Lorsque X ou Y représente un radical amino substitué par un ou deux radicaux alkyles, ceux-ci sont de préférence des radicaux méthyle ou éthyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques.

L'invention concerne notamment des composés de formule (I) caractérisés en ce que X représente un radical méthoxy, nitro, amino ou acétylamino et Y représente un atome d'hydrogène ou bien Y représente un atome de chlore, un radical nitro, amino ou acétylamino et X représente un atome d'hydrogène, ainsi que leurs sels d'addition avec les acides et tout particulièrement le 2-[3-[(1,1-diméthyléthyl) amino] 2-hydroxy propoxy]N-(1H-indol-4-yl) 5-nitro benzamide ainsi que ses sels d'addition avec les acides.

Les composés de formule (I) de la présente invention peuvent être préparés par un procédé semblable à celui décrit dans la demande de brevet européen n° 86401528.4.

Le procédé de préparation des produits de la présente demande est caractérisé en ce que l'on fait réagir un composé de formule (II) :

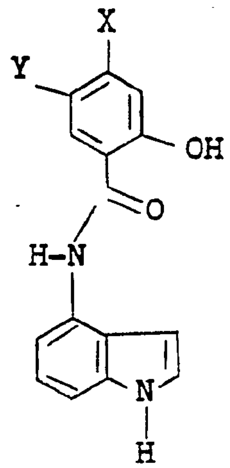

(II)

dans laquelle X et Y ont les significations précédentes, avec un halogénure de formule (III) :

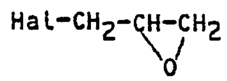

(III)

pour obtenir un composé de formule (IV) :

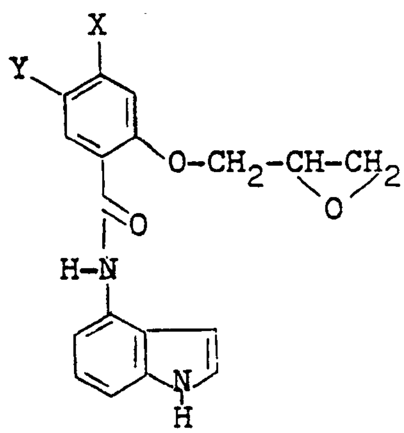

IV

que l'on fait réagir avec la ter-butylamine pour obtenir un composé de formule (I) que, si désiré, on soumet à l'action d'un acide minéral ou organique pour obtenir un sel.

Lorsque X ou Y représente un groupement alkyle, alkoxy ou un atome de chlore, de brome ou d'iode dans le composé de formule (II), Hal est de préférence un atome de chlore dans le composé de formule (III).

La réaction du composé de formule (II) avec le composé de formule (III) est alors de préférence réalisée en présence d'une base telle que le carbonate de potassium ou de sodium, la soude ou la potasse.

Lorsque X ou Y représente un groupement nitro dans le composé de formule (II), Hal est de préférence un atome de brome dans le composé de formule (III). La réaction est réalisée alors en chauffant au reflux le composé de formule (IV).

La réaction du dérivé de formule (IV) avec la terbutylamine est réalisée en utilisant un solvant tel qu'un alcool aliphatique comme le méthanol ou l'éthanol.

Lorsque l'on désire préparer un composé de formule (I) dans laquelle X ou Y représente un radical amino éventuellement substitué, on peut réduire le composé de formule (I) dans laquelle X ou Y représente un radical nitro et le cas échéant, on fait réagir le dérivé amino avec un dérivé du substituant que l'on désire obtenir.

Les conditions opératoires correspondantes sont connues de l'homme de métier.

Le produit de départ de formule (II) peut être préparé par réaction du 4-amino indole avec un acide hydroxy benzoïque substitué par X ou Y.

Les dérivés, objet de la présente invention, possèdent de très intéressantes propriétés pharmacologiques; ils sont doués notamment de remarquables propriétés antiarythmiques et certains produits possèdent des propriétés anticalciques.

Ces propriétés sont illustrées plus loin dans la partie expérimentale.

Ces propriétés justifient l'utilisation des dérivés du N(1H-indol-4-yl) benzamide de formule (I), ainsi que de leurs sels, à titre de médicaments.

La présente demande a ainsi également pour objet l'application, à titre de médicaments, des dérivés du N-(1H-indol-4-yl) benzamide, tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du N-(1H-indol-4-yl) benzamide répondant à la formule (I), caractérisés en ce que X représente un radical méthoxy, nitro, amino ou acétylamino et Y représente un atome d'hydrogène ou bien Y représente un atome de chlore, un radical nitro, amino ou acétylamino et X représente un atome d'hydrogène ainsi que leurs sels d'addition avec les acides.

Parmi ceux-ci, on retient tout particulièrement le 2-[3-[(1,1-diméthyléthyl)amino] 2-hydroxy propoxy] N-(1H-indol-4-yl) 5-nitro benzamide ainsi que ses sels d'addition avec les acides.

Les médicaments selon l'invention trouvent leur emploi, par exemple dans le traitement des arythmies.

La dose usuelle, variable selon le dérivé utilisé, le sujet et l'affection en cause peut être par exemple, de 50 mg à 1 g par jour. Par voie orale, chez l'homme, le dérivé de l'exemple 1 peut être administré à la dose quotidienne de 200 mg à 800 mg, par exemple pour le traitement des arythmies ventriculaires, supraventriculaires et jonctionnelles, soit environ de 3 mg à 12 mg par kilogramme de poids corporel.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples qui suivent illustrent la présente invention sans toutefois la limiter.

Exemple 1 : 2-[3[(1,1-diméthyléthyl) amino] 2-hydroxy propoxy] N-(1H-indol-4-yl) 5-nitro benzamide et son chlorhydrate.

On chauffe au reflux pendant 1 heure 9,8 g de 2-hydroxy 5-nitro N-(1H-indol-4-yl) benzamide avec 40 cm3 d'épibromhydrine. On élimine sous pression réduite à 60°C l'excès d'épibromhydrine, reprend la résine obtenue par 100 cm3 d'éthanol et 40 cm3 de terbutylamine et chauffe au reflux pendant 2 heures. On élimine l'excès de solvant et de réactif sous pression réduite à 60°C et obtient 78 g de produit brut. La purification s'effectue par deux chromatographies successives sur silice (1ère : éluant : chloroforme 8-méthanol 1-triéthylamine 1, 2ème : éluant : chloroforme 5-acétone 4-triéthylamine 1). On récupère 4,4 g de produit sous forme de base F = 208°C.

Formation du chlorhydrate

On dissout 3,5 g de base préparée précédemment dans un litre d'isopropanol et 250 cm3 de méthanol au reflux, ajoute une solution saturée d'acide chlorhydrique dans l'acétate d'éthyle jusqu'à pH acide. On chauffe 30 minutes au reflux, concentre jusqu'à 500 cm3 environ, glace, filtre et sèche sous pression réduite à 50°C. On obtient 2,85 g de produit attendu. F > 260°C.

Analyse :

Calculé : C% 57,08 H% 5,88 N% 12,10 Cl% 7,66
Trouvé : C% 56,8 H% 6,1 N% 12,0 Cl% 7,4

Préparation du 2-hydroxy 5-nitro N-(1H-indol-4-yl) benzamide

On chauffe au reflux pendant 3 heures, 6 g de 4-amino indole, 150 cm3 de tétrahydrofuranne, 8,4 g d'acide 5-nitro salicylique et 12,5 g de dicyclohexylcarbodiimide. On refroidit, filtre, ajoute 200 cm3 d'acétate d'éthyle, élimine l'excès de 4-amino indole n'ayant pas réagi par lavage avec de l'acide chlorhydrique 1N, sèche, filtre, évapore à sec sous pression réduite à 50°C. On obtient 20 g d'une résine que l'on empâte par un mélange chloroforme méthanol (50/50), filtre, sèche sous pression réduite et obtient 7,5 g de produit attendu F > 260°C.

Exemple 2 : 5-amino 2-[3-[(1,1-diméthyléthyl)amino] 2-hydroxy propoxy] N-(1H-indol-4-yl) benzamide.

On chauffe 1 heure au reflux, 142 mg de produit de l'exemple 1,5 cm3 de méthanol, une pincée de nickel de Raney et 0,2 cm3 d'hydrate d'hydrazine. On filtre, élimine le méthanol sous pression réduite à 50°C et obtient 128 mg de produit attendu.

Spectre UV :

éthanol :
Max. 219 nm $E_1^1 = 443$
Max. 310 nm $E_1^1 = 230$
en éthanol = $HCl\frac{N}{10}$
Max. 299 nm $E_1^1 = 208$ $\epsilon = 8200$
Infl. : 275 et 332 nm

Exemple 3 : 5-acétylamino 2-[3-[1,1-diméthyléthyl) amino] 2-hydroxy propoxy] N-(1H-indol-4-yl) benzamide et son oxalate neutre.

Stade A : 5-amino 2-hydroxy N-(1H-indol-4-yl) benzamide.

On chauffe au reflux pendant 2 heures une suspension comprenant 6 g de 5-nitro 2-hydroxy N-(1H-indol-4-yl) benzamide préparé à l'exemple 1, 600 cm3 de méthanol et 15 cm3 d'hydrate d'hydrazine à 64% en présence de 6 g de Nickel de Roney. On filtre la solution obtenue, élimine le solvant sous pression réduite à 50°C, reprend le résidu par un mélange chloroforme-méthanol (1-1), filtre le produit cristallisé, sèche et recueille 4 g de produit attendu. F > 260°C.

Stade B : 5-acétylamino 2-acétoxy N-(1H-indol-4-yl) benzamide.

On refroidit à 0/+5°C, 2,6 g de produit préparé au stade précédent, en suspension dans 70 cm3 de tétrahydrofuranne puis ajoute 4,7 cm3 d'anhydride acétique puis maintient 3 heures à température ambiante. On élimine le solvant sous pression réduite à 50°C, reprend le résidu dans un mélange chloroforme-méthanol (1-1), filtre et obtient après séchage à 80°C, 2,7 g de produit attendu. F = 263°C.

Stade C : 5-acétylamino 2-hydroxy N-(1H-indol-4-yl) benzamide.

On ajoute à température ambiante 1,5 g d'hydrure de bore et de sodium dans une suspension comprenant 1,5 g de produit obtenu au stade précédent et 150 cm3 de méthanol, puis chauffe 2 heures au reflux. On élimine partiellement le méthanol sous pression réduite à 50°C, dilue par 200 cm3 d'eau et 200 cm3 d'acétate d'éthyle puis extrait à l'acétate d'éthyle. On sèche, élimine les solvants sous pression réduite à 50°C et recueille 1,35 g de produit attendu utilisé tel quel pour le stade suivant.

Stade D : 5-acétylamino 2-[(2-oxiranyl) méthoxy] N-(1H-indol-4-yl) benzamide.

On chauffe au reflux pendant 2 heures, 3,5 g de produit préparé comme au stade C, 150 cm3 d'acétone, 1,6 g de carbonate de potassium et 9 cm3 d'épibromhydrine. On ajoute de nouveau 9 cm3 d'épibromhydrine et poursuit le reflux pendant 20 heures. On filtre, lave à l'acétone, élimine le solvant sous pression réduite à 50°C, reprend le résidu dans l'éther, filtre, sèche à 80°C et récupère 3,4 g de produit attendu. F = 230°C.

Stade E : 5-acétylamino 2-[3-[1,1-diméthyléthyl) amino] 2-hydroxy propoxy] N-(1H-indol-4-yl) benzamide et son oxalate neutre.

On chauffe au reflux pendant 1 heure, 3,4 g du produit obtenu ci-dessus dans 200 cm3 d'éthanol et 20 cm3 de terbutylamine. On élimine les solvants sous pression réduite à 50°C, chromatographie le résidu sur silice (éluant : chloroforme-méthanol-triéthylamine 8-1-1) et recueille 3,9 g de base attendue.

Préparation de l'oxalate.

On dissout 2,7 g de base dans 200 cm3 d'éthanol et ajoute 330 mg d'acide oxalique. On filtre, sèche à 80°C sous pression réduite et obtient 2,2 g de l'oxalate attendu. F > 260°C.

Analyse :$C_{24}H_{30}N_4O_4$, 1/2 $C_2H_2O_4$ : 423,549

Calculé : C% 62,10 H% 6,46 N% 11,59
Trouvé : C% 61,9 H% 6,2 N% 11,5

Exemple 4 : 2-[3-[1,1-diméthyléthyl) amino] 2-hydroxy propoxy] N-(1H-indol-4-yl) 4-méthoxy benzamide et son benzoate.

Stade A : 4-méthoxy 2-[(2-oxiranyl) méthoxy] N-(1H-indol-4-yl) b enzamide.

On opère comme au stade D de l'exemple 3 à partir de 5 g de 4-méthoxy 2-hydroxy N-(1H-indol-4-yl) benzamide, 150 cm3 d'acétone, 2,45 g de carbonate de potassium et 14 cm3 d'épichlorhydrine. On obtient 7,3 g de produit attendu. F = 157°C.

Stade B : 2-[3-[1,1-diméthyléthyl) amino] 2-hydroxy propoxy] N-(1H-indol-4-yl) 4-méthoxy benzamide et son benzoate.

On opère comme au stade E de l'exemple 3 à partir de 6 g du produit préparé au stade A ci-dessus, 100 cm3 d'éthanol, 9,2 cm3 de terbutylamine, en maintenant le reflux pendant 3 heures. On obtient 6,1 g de base attendue que l'on dissout dans 300 cm3 d'isopropanol au reflux puis ajoute 1,8 g d'acide benzoïque. On concentre partiellement, glace, filtre, sèche sous pression réduite et obtient 5,1 g de benzoate attendu. F = 195°C après recristallisation dans l'éthanol.

Analyse : $C_{23}H_{29}N_3O_4$m $C_7H_6O_2$ : 533,629

Calculé : C% 67,53 H% 6,61 H% 7,87
Trouvé : C% 67,5 H% 6,7 N% 8,0

Préparation du 4-méthoxy 2-hydroxy N-(1H-indol-4-yl) benzamide utilisé au départ de l'exemple 4.

On chauffe au reflux pendant 24 heures 3,96 g de 4-amino 1H-indole, 70 cm3 de tétrahydrofuranne, 5 g d'acide 2-hydroxy 4-méthoxybenzoïque et 6,2 g de dicyclohexylcarbodiimide. On élimine le solvant sous pression réduite à 50°C, reprend le résidu dans l'acétate d'éthyle et élimine le 4-amino indole n'ayant pas réagi par lavage à l'acide chlorhydrique 2N. On élimine l'acétate d'éthyle sous pression réduite, reprend le résidu à l'éther et sèche à 50°C. On obtient 7,1 g de produit attendu. F = 190°C.

Exemple 5 : 5-chloro 2-[3-[1,1-diméthyléthyl) amino] 2-hydroxy propoxy] N-(1H-indol-4-yl) benzamide et son oxalate neutre.

Stade A : 5-chloro 1-[(2-oxiranyl) méthoxy] N-(1H-indol-4-yl) benzamide.

On opère comme au stade D de l'exemple 3 à partir de 3 g de 5-chloro 2-hydroxy N-(1H-indol-4-yl) benzamide, 100 cm3 d'acétone, 1,47 g de carbonate de potassium et 8,3 cm3 d'épichlorhydrine. On obtient 3,35 g de produit attendu. F = 175°C.

Stade B : 5-chloro 2-[3-[1,1-diméthyléthyl) amino] 2-hydroxy propoxy] N-(1H-indol-4-yl) benzamide et son oxalate neutre.

On opère comme au stade E de l'exemple 3 à partir de 3 g du produit obtenu au stade A, 60 cm3 d'éthanol et 4,6 cm3 de terbutylamine. On obtient 2,6 g de base attendue puis 2,4 g d'oxalate. F > 260°C.

Analyse : $C_{22}H_{26}ClN_3O_3m$ 1/2 $C_2H_2O_4$ : 460, 941

Calculé : C% 59,93 H% 5,90 N% 9,12 Cl% 7,69
Trouvé : C% 60,2 H% 5,8 N% 9,1 Cl% 7,9

Préparation du 5-chloro 2-hydroxy N-(1H-indol-4-yl) benzamide utilisé au départ de l'exemple 5.

On chauffe au reflux 3,96 g de 4-amino 1H-indole dans 75 cm3 de tétrahydrofuranne avec 5,16 g d'acide 5-chloro solicyclique et 6,2 g de dicyclohexylcarbodiimide, pendant 2 heures ; on ajoute de nouveau 0,62 g de dicyclohexylcarbodiimide, chauffe encore pendant 1 heure au reflux, refroidit, filtre, élimine le solvant sous pression réduite à 50°C. On reprend le résidu dans l'acétate d'éthyle, lave avec de l'acide chlorhydrique 2N, sèche et élimine l'acétate d'éthyle sous pression réduite à 50°C. Après chromatographie du résidu sur silice (éluant: chloroformeacétate d'éthyle 9-1), on récupère 3,9 g de produit attendu. F = 248°C.

Exemple 6 :

On a préparé des comprimés répondant à la formule :
– produit de l'exemple 1    50 mg
– excipient q.s. pour un comprimé terminé à    100 mg
(détail de l'excipient : lactose, amidon, talc, stéarate de magnésium).

Etude pharmacologique

1/. Action antiarythmique chez le rat :

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse ou par voie orale.

Cinq minutes après l'administration du produit par voie intraveineuse, on perfuse la veine jugulaire des rats avec 10 µg/mn d'une solution d'aconitine et on note le temps d'apparition des troubles du rythme cardiaque (10 µg d'aconitine correspondant à la perfusion de 0,2 ml de solution).

Les résultats sont exprimés en pourcentage d'allongement du temps d'apparition des troubles du rythme cardiaque par rapport aux témoins et en fonction de la dose du produit testé. Les résultats figurant sur le tableau ci-après montrent que les produits de la présente demande sont doués de remarquables propriétés antiarythmiques.

| Produit de l'exemple voie I.V. voie | Dose en mg/kg | Pourcentage d'allongement du temps |
|---|---|---|
| 1 | 2,5 | +77 % |
| | 1 | +39 % |
| | 0,5 | +17% |
| 4 | 2,5 | +38% |
| | 1 | +10% |

2/. Test d'activité anticalcique in vitro.

Des artères caudales de rat découpées en spirale sont reliées à des capteurs de tension et sont maintenues dans des cuves de 25 ml de tampon Krebs-bicarbonate de sodium (NaCl : 120,8 mM, KCl : 5,9 mM, $MgCL_2$ : 1,2 mM, $NaH_2PO_4$ : 1,2 mM, $NaHCO_3$ : 15,5 mM, glucose 12,6 mM) à 37°C gazée avec un mélange $O_2$ : 95%–$CO_2$ 5%.

Les préparations sont dépolarisées par une solution tampon à concentration 100 mM en ions $K^+$ (NaCl : 26,7 mM, KCl : 100 mM, $MgCl_2$ : 1,2 mM, $NaH_2PO_4$ : 1,2 mM, $NaHCO_3$ 15,5 mM, glucose : 12,6 mM).

On ajoute, sous un volume de 250 ul, du chlorure de calcium, de manière à obtenir une gamme de concentrations croissantes en ions $Ca^{2+}$ allant de 0,1 à 3,0 mM ; on enregistre les contractions des artères et établit ainsi une gamme témoin. On répète l'opération avec la gamme d'ions $Ca^{2+}$ toutes les 15 minutes et la préparation est lavée quatre fois, après chaque gamme.

Lorsque l'on obtient une réponse stable, l'on effectue l'opération avec les gammes d'ions $Ca^{2+}$ en présence de différentes concentrations du produit à tester, jusqu'à ce qu'une réponse stable soit obtenue.

Les contractions des artères dépendent de l'entrée des ions $Ca^{2+}$ dans les cellules des muscles lisses et sont provoquées par la dépolarisation du muscle lisse par les ions $K^+$ et par l'action de la noradrénaline libérée au niveau présynaptique. En recommançant l'opération avec des artères dénervées par action de 6-OH dopamine, on supprime l'action propre due à la noradrénaline.

Les résultats sont exprimés en CI 50 (concentration inhibitrice 50) concentration du produit testé qui inhibe de 50% la contraction due aux ions $K^+$.

On constate d'après les résultats figurant sur le tableau ci-après que les produits de la présente demande possèdent une forte activité anticalcique.

| Produit de l'exemple | CI 50 en µM |
|---|---|
| 4 | 8 |
| 5 | 5,4 |

3/. Etude de la toxicité aigüe :

On a évalué la dose létale $DL_0$ du composé de l'exemple 1 testé après administration par voie orale chez la souris.

On appelle $DL_0$, la dose maximale ne provoquant aucune mortalité en 7 jours.

La $DL_0$ trouvée est de 200 mg/Kg.

**Revendications**

**Revendications pour les Etats Contractants : AT, BE, CH, DE, FR, GB, IT, LI, LU, NL, SE.**

1. Composés de formule (I) :

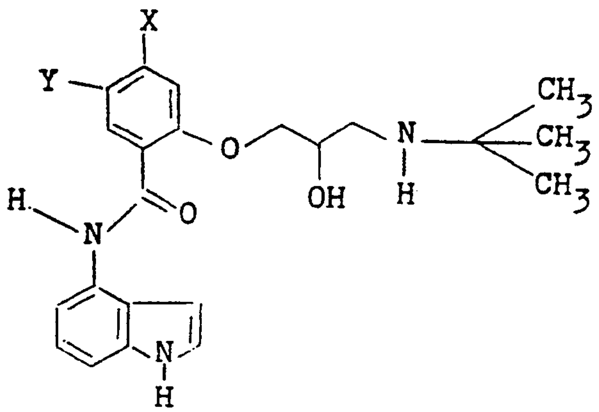

(I)

dans laquelle l'un ou l'autre des substituants X ou Y représente un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou un ou deux radicaux alkyles renfermant de 1 à 5 atomes de carbone et l'autre substituant représente un atome d'hydrogène, étant entendu que Y ne peut pas être le groupement méthoxy et que X ne peut pas être un atome de chlore ainsi que leurs sels d'addition avec les acides.

2. Composés de formule (I), tels que définis à la revendication 1, caractérisés en ce que X représente un radical méthoxy, nitro, amino ou acétylamino et Y représente un atome d'hydrogène ou bien Y représente un atome de chlore, un radical nitro, amino ou acétylamino et X représente un atome d'hydrogène, ainsi que leurs sels d'addition avec les acides.

3. Le 2-[3-[(1,1-diméthyléthyl) amino] 2-hydroxy propoxy] N-(1H-indol 4-yl) 5-nitro benzamide ainsi que ses sels d'addition avec les acides.

4. Procédé de préparation des produits de formule (I), caractérisé en ce que l'on fait réagir un composé de formule (II) :

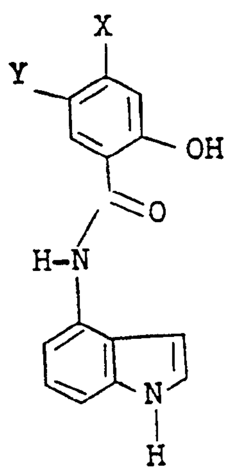

(II)

dans laquelle X et Y ont les significations précédentes, avec un halogénure de formule (III) :

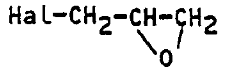

(III)

pour obtenir un composé de formule (IV) :

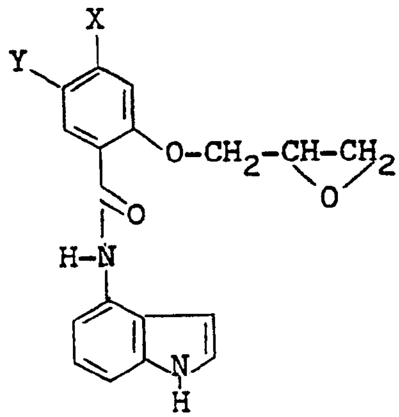

(IV)

que l'on fait réagir avec la terbutylamine pour obtenir un composé de formule (I) que, si désiré, on soumet à l'action d'un acide minéral ou organique pour obtenir un sel.

5. Médicaments, caractérisés en ce qu'ils sont constitués par les dérivés du N-(1H-indol-4-yl) benzamide, tels que définis par la formule (I) de la revendication 1, ainsi que par leurs sels d'addition avec les acides pharmaceutiquement acceptables.

6. Médicaments, caractérisés en ce qu'ils sont constitués par les dérivés du N-(1H-indol 4-yl) benzamide, tels que définis dans la la revendication 2.

7. Médicaments, caractérisés en ce qu'ils sont constitués par les dérivés du N-(1H-indol-4-yl) benzamide, tels que définis dans la la revendication 3.

8. Compositions pharmaceutiques, caractérisés en ce qu'elles renferment à titre de principe actif, l'un au moins des médicaments défini à l'une des revendications 5 à 7.

**Revendications pour les Etats contractants suivants : ES, GR**

1. Procédé de préparation des dérivés du N-(1H-indol-4-yl) benzamide ainsi que leurs sels d'addition avec les acides, répondant à la formule (I) :

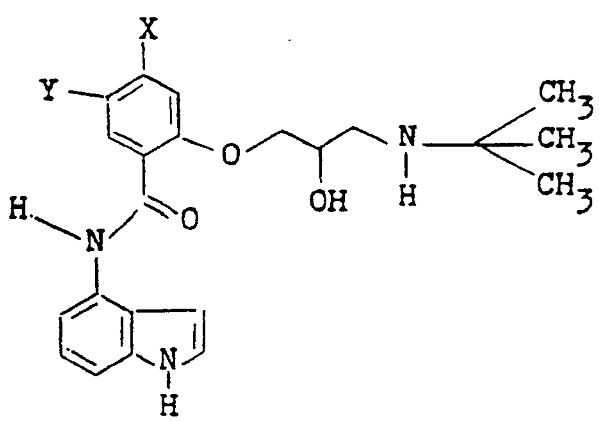

(I)

dans laquelle l'un ou l'autre des substituants X ou Y représente un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou un ou deux radicaux alkyles renfermant de 1 à 5 atomes de carbone et l'autre

substituant représente un atome d'hydrogène, étant entendu que Y ne peut pas être le groupement méthoxy et que X ne peut pas être un atome de chlore, caractérisé en ce que l'on fait réagir un composé de formule (II) :

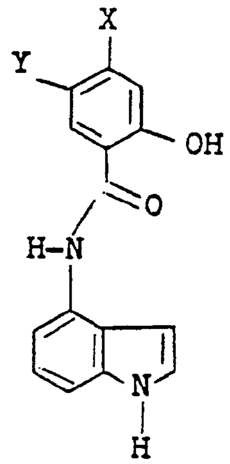

(II)

dans laquelle X et Y ont les significations précédentes, avec un halogénure de formule (III) :

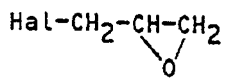

(III)

pour obtenir un composé de formule (IV) :

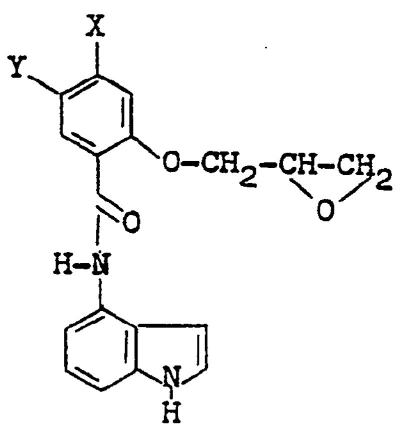

(IV)

que l'on fait réagir avec la terbutylamine pour obtenir un composé de formule (I) que, si désiré, on soumet à l'action d'un acide minéral ou organique pour obtenir un sel.

2. Procédé selon la revendication 1, caractérisé en ce que l'on utilise au départ un produit de formule (II) dans laquelle X représente un radical méthoxy, nitro, amino ou acétylamino et Y représente un atome d'hydrogène ou bien Y représente un atome de chlore, un radical nitro, amino ou acétylamino et X représente un atome d'hydrogène.

## Ansprüche

### Patentansprüche für die Vertragsstaaten : AT, BE, CH, DE, FR, GB, IT, LI, LU, NL, SE

1. Verbindungen der Formel (I)

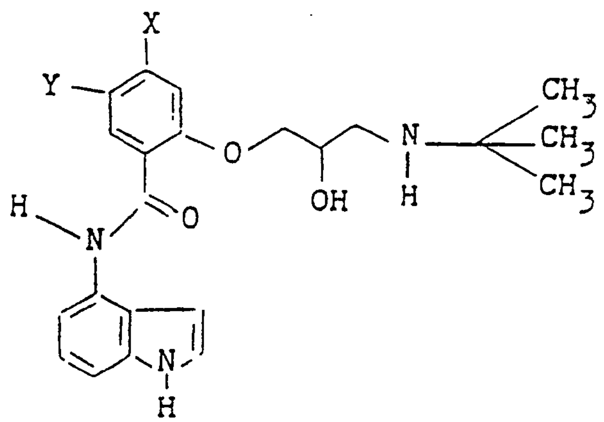

(I)

worin der eine oder andere der Substituenten X oder Y einen Alkylrest mit 1 bis 5 Kohlenstoffatomen, einen Alkoxyrest mit 1 bis 3 Kohlenstoffatomen, ein Chlor-, Brom- oder Jodatom, einen Nitrorest oder einen Aminorest, gegebenenfalls substituiert durch einen aliphatischen Acylrest mit 2 bis 5 Kohlenstoffatomen oder ein oder zwei Alkylreste mit 1 bis 5 Kohlenstoffatomen, bedeuten und der andere Substituent ein Wasserstoffatom darstellt, wobei Y nicht die Methoxygruppe und X nicht ein Chloratom sein kann, sowie ihre Säureadditionssalze.

2. Verbindungen der Formel (I), wie sie in Anspruch 1 definiert sind, dadurch gekennzeichnet, daß X einen Methoxy-, Nitro-, Amino- oder Acetylaminorest bedeutet, und Y ein Wasserstoffatom darstellt, oder aber Y ein Chloratom, einen Nitro-, Amino- oder Acetylaminorest bedeutet und X ein Wasserstoffatom darstellt, sowie ihre Säureadditionssalze.

3. Das 2-[3-[(1,1-Dimethylethyl)-amino]-2-hydroxy-propoxy]-N-(1H-indol-4-yl)-5-nitrobenzamid sowie seine Säureadditionssalze.

4. Verfahren zur Herstellung der Produkte der Formel (I), dadurch gekennzeichnet, daß man eine Verbindung der Formel (II)

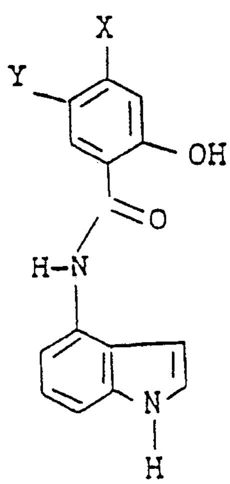

(II)

worin X und Y die vorstehenden Bedeutungen haben, mit einem Halogenid der Formel (III) :

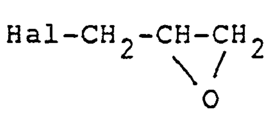

(III)

zur Reaktion bringt, um eine Verbindung der Formel (IV)

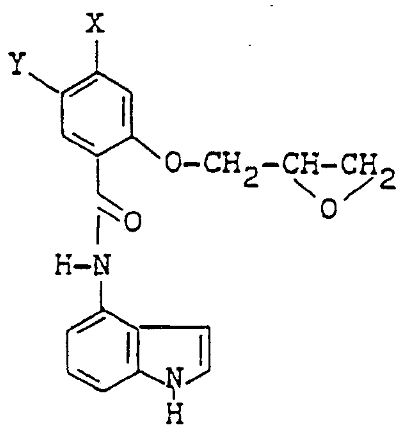

(IV)

zu erhalten, die man mit tert.-Butylamin umsetzt, um eine Verbindung der Formel (I) zu erhalten, die man gewünschtenfalls der Einwirkung einer Mineral- oder organischen Säure unterwirft, um ein Salz zu erhalten.

5. Arzneimittel, dadurch gekennzeichnet, daß sie aus Derivaten des N-(1H-Indol-4-yl)-benzamids, wie in Formel (I) des Anspruchs 1 definiert, sowie aus ihren Additionssalzen mit pharmazeutisch annehmbaren Säuren bestehen.

6. Arzneimittel, dadurch gekennzeichnet, daß sie aus den Derivaten des N-(1H-Indol-4-yl)-benzamids, wie in Anspruch 2 definiert, bestehen.

7. Arzneimittel, dadurch gekennzeichnet, daß sie aus den Derivaten des N-(1H-Indol-4-yl)-benzamids, wie in Anspruch 3 definiert, bestehen.

8. Pharmazeutische Zusammensetzungen, dadurch gekennzeichnet, daß sie als aktives Prinzip mindestens eines der Arzneimittel, wie in einem der Ansprüche 5 bis 7 definiert, enthalten.

**Patentansprüche für die Vertragsstaaten : ES, GR**

1. Verfahren zur Herstellung von Derivaten des N-(1H-Indol-4-yl)-benzamids sowie von deren Additionssalzen mit Säuren,entsprechend der Formel (I) :

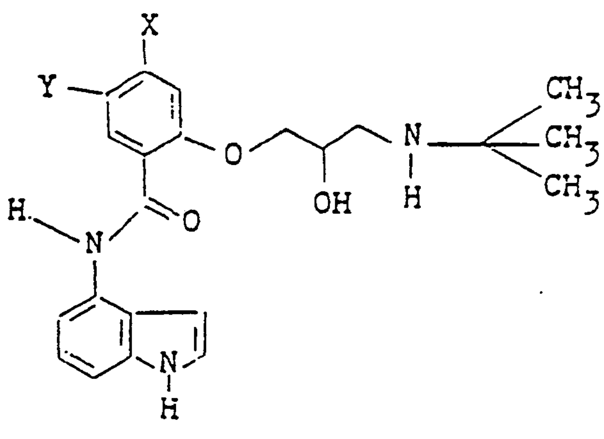

(I)

worin der eine oder andere der Substituenten X oder Y einen Alkylrest mit 1 bis 5 Kohlenstoffatomen, einen Alkoxyrest mit 1 bis 3 Kohlenstoffatomen, ein Chlor-, Brom- oder Jodatom, einen Nitrorest oder einen Aminorest, gegebenenfalls substituiert durch eine aliphatische Acylgruppe mit 2 bis 5 Kohlenstoffatomen, oder ein oder zwei Alkylreste mit 1 bis 5 Kohlenstoffatomen, bedeuten und der andere Substituent ein Wasserstoffatom darstellt, wobei Y nicht die Methoxygruppe und X nicht ein Chloratom sein kann, dadurch gekennzeichnet, daß man eine Verbindung der Formel (II)

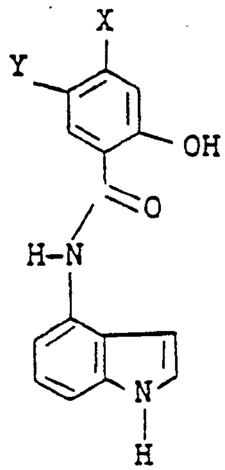

(II)

worin X und Y die vorstehenden Bedeutungen haben, mit einem Halogenid der Formel (III)

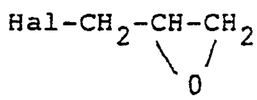

(III)

zur Reaktion bringt, um eine Verbindung der Formel (IV)

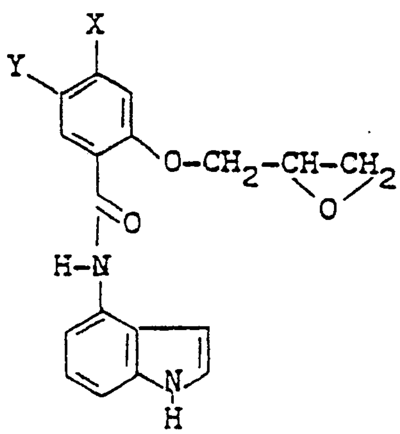

(IV)

zu erhalten, die man mit tert.-Butylamin umsetzt, um eine Verbindung der Formel (I) zu erhalten, die man gewünschtenfalls falls der Einwirkung einer Mineral- oder organischen Säure unterwirft, um ein Salz zu erhalten.

2. Verfahren gemäß Anspruch 1, dadurch gekennzeichnet, daß man als Ausgangsstoff ein Produkt der Formel (II) verwendet, worin X einen methoxy-, Nitro-, Amino- oder Acetylaminorest bedeutet und Y ein Wasserstoffatom bedeutet, oder aber Y ein Chloratom, einen Nitro-, Amino- oder Acetylaminorest bedeutet und X ein Wasserstoffatom bedeutet.

## Claims

### Claims for the contracting states : AT, BE, CH, DE, FR, GB, IT, LI, LU, NL, SE

1. Compounds of formula (I) :

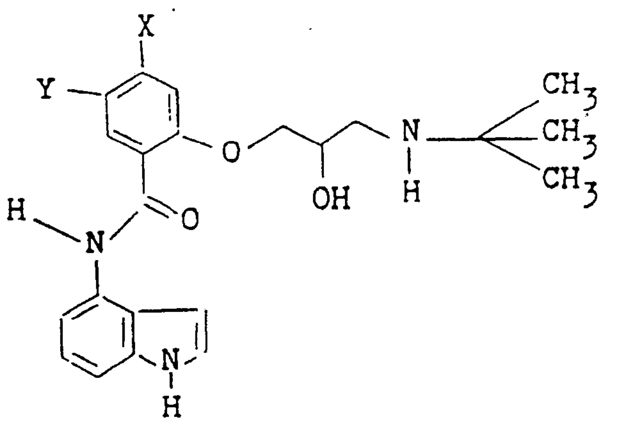

(I)

in which one or other of the substituents X or Y represents an alkyl radical containing 1 to 5 carbon atoms, an alkoxy radical containing 1 to 3 carbon atoms, a chlorine, bromine or iodine atom, a nitro radical or an amino radical optionally substituted by an aliphatic acyl group containing 2 to 5 carbon atoms or one or two alkyl radicals containing 1 to 5 carbon atoms and the other substituent represents a hydrogen atom, it being understood that Y cannot be the methoxy group and that X cannot be a chlorine atom, as well as their addition salts with acids.

2. Compounds of formula (I), as defined in claim 1, characterized in that X represents a methoxy, nitro, amino or acetylamino radical and Y represents a hydrogen atom or Y represents a chlorine atom, a nitro, amino or acetylamino radical and X represents a hydrogen atom, as well as their addition salts with acids.

3. 2-[3-[(1,1-dimethylethyl)-amino]-2-hydroxy-propoxy]-N-(1H-indol-4-yl)-5-nitro benzamide, as well as its addition salts with acids.

4. Preparation process for products of formula (I), characterized in that a compound of formula (II) :

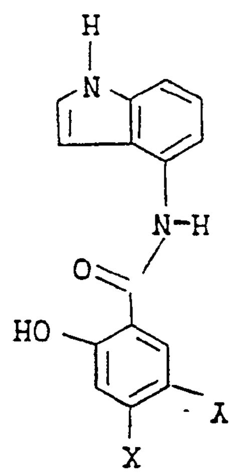

(II)

in which X and Y have the previous meanings, is reacted with a halide of formula (III) :

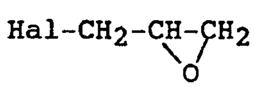

(III)

to obtain a compound of formula (IV) :

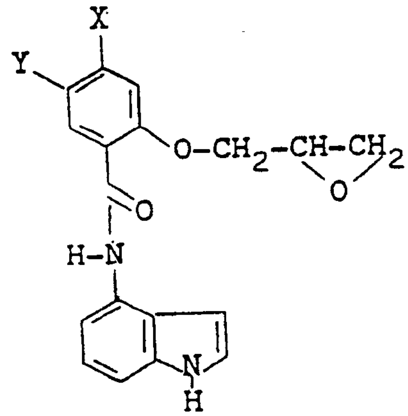

(IV)

which is reacted with tertbutylamine to obtain a compound of formula (I) which, if desired, is subjected to the action of a mineral or organic acid to obtain a salt.

5. Medicaments, characterized in that they are constituted by the derivatives of N-(1H-indol-4-yl)-benzamide, as defined by the formula (I) of claim 1, as well as by their addition salts with pharmaceutically acceptable acids.

6. Medicaments, characterized in that they are constituted by the derivatives of N-(1H-indol-4-yl)-benzamide, as defined in claim 2.

7. Medicaments, characterized in that they are constituted by the derivatives of N-(1H-indol-4-yl)-benzamide, as defined in claim 3.

8. Pharmaceutical compositions, characterized in that they contain as active ingredient, at least one of the medicaments defined in one of claims 5 to 7.

**Claims for the contracting states : ES, GR**

1. Preparation process for derivatives of N-(1H-indol-4-yl)-benzamide as well as their addition salts with acids, corresponding to the formula (I) :

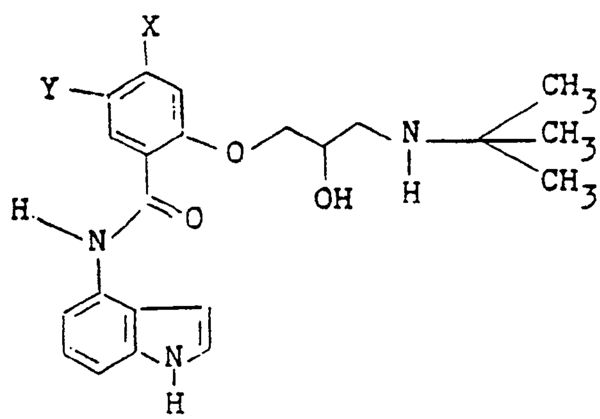

(I)

in which one or other of the substituents X or Y represents an alkyl radical containing 1 to 5 carbon atoms, an alkoxy radical containing 1 to 3 carbon atoms, a chlorine, bromine or iodine atom, a nitro radical or an amino radical optionally substituted by an aliphatic acyl group containing 2 to 5 carbon atoms or one or two alkyl radicals containing 1 to 5 carbon atoms and the other substituent represents a hydrogen atom, it being understood that Y cannot be the methoxy group and that X cannot be a chlorine atom, characterized in that a compound of formula (II) :

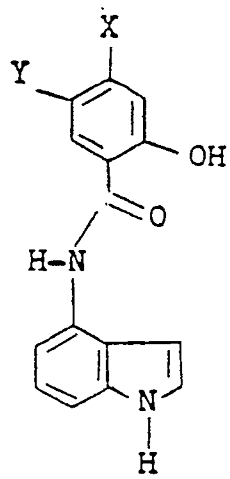

(II)

in which X and Y have the previous meanings, is reacted with a halide of formula (III) :

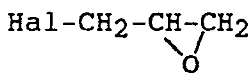

(III)

to obtain a compound of formula (IV) :

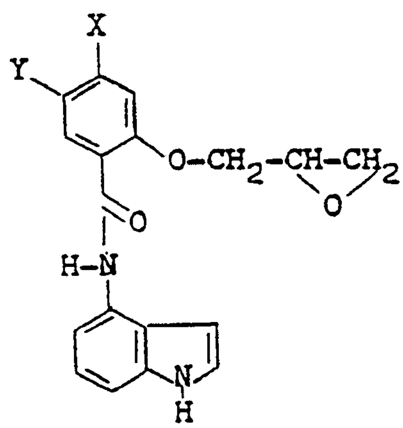

(IV)

which is reacted with tertbutylamine to obtain a compound of formula (I) which, if desired, is subjected to the action of a mineral or organic acid to obtain a salt.

2. Process according to claim 1, characterized in that at the start a product of formula (II) is used in which X represents a methoxy, nitro, amino or acetylamino radical and Y represents a hydrogen atom or Y represents a chlorine atom, a nitro, amino or acetylamino radical and X represents a hydrogen atom.